(19) Europäisches Patentamt / European Patent Office / Office européen des brevets

(11) **EP 2 532 757 B1**

(12) **EUROPEAN PATENT SPECIFICATION**

(45) Date of publication and mention of the grant of the patent:
**21.10.2015 Bulletin 2015/43**

(51) Int Cl.:
***C12Q 1/68*** (2006.01)

(21) Application number: **11382187.0**

(22) Date of filing: **06.06.2011**

(54) **In vitro method for predicting fecundity of semen**

In-vitro-Verfahren zur Vorhersage der Fruchtbarkeit von Samen

Procédé in vitro de prédiction de la fécondité du sperme

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**12.12.2012 Bulletin 2012/50**

(73) Proprietors:
- **Institut d'Investigació Biomèdica de Bellvitge (IDIBELL)**
  **08908 l'Hospitalet de Llobregat (ES)**
- **Fundació Puigvert**
  **08025 Barcelona (ES)**

(72) Inventors:
- **Larriba Bartolomé, Sara**
  **08028 Barcelona (ES)**
- **Lluís Bassas, Arnau**
  **08911 Badalona (ES)**

(74) Representative: **Illescas, Manuel et al**
**Manuel Illescas y Asociados, S.L. (MIA)**
**Principe de Vergara n° 197**
**Oficina 1°A**
**28002 Madrid (ES)**

(56) References cited:
**FR-A1- 2 721 405**

- **C. LALANCETTE ET AL:** "Transcriptome Analysis of Bull Semen with Extreme Nonreturn Rate: Use of Suppression-Subtractive Hybridization to Identify Functional Markers for Fertility", BIOLOGY OF REPRODUCTION, vol. 78, no. 4, 1 April 2008 (2008-04-01), pages 618-635, XP55005302, ISSN: 0006-3363, DOI: 10.1095/biolreprod.106.059030
- **FEUGANG J M ET AL:** "Transcriptome analysis of bull spermatozoa: implications for male fertility", REPRODUCTIVE BIOMEDICINE ONLINE, REPRODUCTIVE HEALTHCARE LTD, GB, vol. 21, no. 3, 1 September 2010 (2010-09-01), pages 312-324, XP027238021, ISSN: 1472-6483 [retrieved on 2010-06-20]
- **MARY K SAMPLASKI ET AL:** "New generation of diagnostic tests for infertility: Review of specialized semen tests", INTERNATIONAL JOURNAL OF UROLOGY, vol. 17, no. 10, 1 October 2010 (2010-10-01), pages 839-847, XP55004033, ISSN: 0919-8172, DOI: 10.1111/j.1442-2042.2010.02619.x
- **OSTERMEIER G C ET AL:** "Spermatozoal RNA profiles of normal fertile men", THE LANCET, LANCET LIMITED. LONDON, GB, vol. 360, no. 9335, 7 September 2002 (2002-09-07), pages 772-777, XP004792312, ISSN: 0140-6736, DOI: DOI:10.1016/S0140-6736(02)09899-9
- **NATHALIE BISSONNETTE ET AL:** "Spermatozoal transcriptome profiling for bull sperm motility: a potential tool to evaluate semen quality", REPRODUCTION, BIOSCIENTIFICA LTD, GB, vol. 138, no. 1, 1 July 2009 (2009-07-01), pages 65-80, XP002638021, ISSN: 1470-1626, DOI: DOI:10.1530/REP-08-0503 [retrieved on 2009-05-07]

- I. GILBERT ET AL: "A molecular analysis of the population of mRNA in bovine spermatozoa", REPRODUCTION, vol. 133, no. 6, 1 June 2007 (2007-06-01), pages 1073-1086, XP55003982, ISSN: 1470-1626, DOI: 10.1530/REP-06-0292
- YATSENKO A N ET AL: "Non-invasive genetic diagnosis of male infertility using spermatozoal RNA: KLHL10 mutations in oligozoospermic patients impair homodimerization", HUMAN MOLECULAR GENETICS, OXFORD UNIVERSITY PRESS, SURREY, vol. 15, no. 23, 1 December 2006 (2006-12-01), pages 3411-3419, XP008088288, ISSN: 0964-6906, DOI: DOI:10.1093/HMG/DDL417
- MILLER D ET AL: "The controversy, potential and roles of spermatozoal RNA", TRENDS IN MOLECULAR MEDICINE, ELSEVIER CURRENT TRENDS, GB, vol. 11, no. 4, 1 April 2005 (2005-04-01), pages 156-163, XP004848183, ISSN: 1471-4914, DOI: DOI:10.1016/J.MOLMED.2005.02.006
- GREENBAUM DOV ET AL: "Interrelating different types of genomic data, from proteome to secretome: 'Oming in on function", GENOME RESEARCH, COLD SPRING HARBOR LABORATORY PRESS, WOODBURY, NY, US, vol. 11, no. 9, 1 September 2001 (2001-09-01), pages 1463-1468, XP009088703, ISSN: 1088-9051, DOI: 10.1101/GR.207401

**Description**

**FIELD OF THE INVENTION**

The present invention may be included in the field of the medicine in general, more particularly in the field of reproductive medicine which comprises the study of the processes involved in reproduction, particularly male and female infertility.

Thus the present invention is focused on the evaluation and assessment of the sperm expression gene profile, in order to identify a specific group of genes whose expression profile is correlated with the fecundity ability of spermatozoa. This group of genes could be used as biomarkers for discriminating those samples with the worst ability to fertilize oocyte.

**STATE OF THE ART**

Traditionally, selection of sperm donors has relied on microscopic assessment to determine semen quality. The assessment of male fertility is based on the descriptive information provided by the basic semen analysis, including sperm count, motility and morphology. New threshold values for semen parameters have been recently updated (Cooper *et al.,* 2010) using men who had produced recent pregnancy as reference individuals. However, these basic semen parameters do not reflect the sperm function and their clinical value in predicting fertility is questionable. Although there is a clear correlation between the semen quality and the probability of conception (Guzick *et al.,* 2001), the wide overlap of parameters between fertile and infertile men suggest that this basic semen analysis (mainly based on sperm count, motility and morphology) does not always correlate with a successful fecundity, and seriously hampers the diagnostic power of semen analysis (Bartoov *et al.,* 1993). The significant proportion of couples having unexplained infertility, in spite of the good quality of the semen, suggests that abnormal sperm function can be due to molecular defects in some cases (Lewis, 2007). Many efforts have been devoted to build up new diagnostic tests to provide more accurate information on the fecundity potential of human spermatozoa (Samplaski *et al.,* 2010) but none of them have yet met the requirements to be adopted for clinical purposes.

Spermatozoa contain, besides the haploid genetic material, an abundant number of functionally viable transcripts (Krawetz, 2005). SAGE and cDNA microarrays on spermatozoa have identified between 3000-7000 different transcripts (Ostermeier *et al.,* 2002; Zhao *et al.,* 2006), commonly considered as remnants of stored mRNA from post-meiotically active genes reflecting the accurate development of spermatogenesis (review in (Miller & Ostermeier, 2006)). The potential for an active post-meiotic production of transcripts, however, exists: a persistence of a low but detectable level of transcription in mature sperm cells was later described (Miteva *et al.,* 1995) and more recently it has been by detection of transcriptional and translational activities, determined in human sperm during capacitation and acrosome reaction (Gur & Breitbart, 2006; Naz, 1998) . Moreover, these stored mRNAs were suggested to be used during the first steps of fertilization, which are not sensitive to transcriptional inhibitors, and then contribute to the paternal printing (Braude *et al.,* 1988; Siffroi & Dadoune, 2001). Ostermeier et al (Ostermeier *et al.,* 2004) reported for the first time that human spermatozoa can deliver mRNA to the oocyte during fertilization. Some of these mRNAs have been described to be translated *de novo* in oocyte after fertilization, thus supporting the hypothesis that, at least some transcripts, might have a function during or beyond the process of fertilization (Gur & Breitbart, 2006) and additionally contribute to the early transcriptome of the embryo (Boerke *et al.,* 2007).

Sperm mRNAs present in the ejaculated spermatozoa have been suggested to represent a genetic fingerprint, and could be considered a historical record of what happened in gene expression during spermatogenesis (Zhao *et al.,* 2006). Some studies have reported differences in the amount of certain sperm transcripts between different groups of men. A decreased *PRM1*/*PRM2* ratio (Steger *et al.,* 2008) as well as *PSG1* and *HLA-E* mRNA levels (Avendano *et al.,* 2009) were observed in infertile compared to fertile men. The *DEAD box polypeptide 4 (VASA)* mRNA expression was higher in normozoospermic than in oligozoospermic men (Guo *et al.,* 2007). A different expression signature or fingerprint was also determined (related to the differences of sperm motility) between normozoospermic and teratozoospermic men (Platts *et al.,* 2007). Interestingly, differences in expression of a few hundreds of transcripts between fertile and infertile men with normal semen parameters using microarray analysis have been recently described (Garrido *et al.,* 2009).

Lalancette and colleagues (Lalancette C. et al. Biol Reprod. 2008;78(4):618-35) evaluated the differences in RNA profiles among spermatozoa from fertile bulls with extreme non-return rates (NRRs): a low-fertile group and a high-fertile group. This measure is defined as the percentage of cows that were inseminated and not re-inseminated within a specified interval, typically 56 days. The NRR is the result of conception and therefore is called the field fertility measure. Using the suppression-subtractive hybridization technique in combination with microarray analysis, genes which are differently expressed are identified (Tables 1 and 2) and the data obtained were validated by quantitative RT-PCT. Moreover, Feugang and colleagues (Feugang JM. Et al. Reprod Biomed Online. 2010;21(3):312-24) analysed the RNA profiles of spermatozoa from the high-fertility and low-fertility Holstein bulls by Afymetrix bovine genechips. Quantitative real-time PCR was used to validate the microarray data and the specific transcripts in spermatozoa could be associated with bulls fertility. It is important to note that these documents refers to bulls, so should be carefully interpreted and not be immediately extrapolated to human and/or other animal being.

Assisted reproduction techniques (ART) have revolutionized the treatment of infertile couples. Among them, therapeutic donor insemination (TDI) provides an ideal first approach to achieve pregnancy in couples with a clear severe male infertility factor. After artificial insemination treatment with sperm donors, differences in pregnancy rates are observed among those donors, although all of them present normal parameters of semen quality. Additional studies suggest that some characteristics of the thawed samples used in the insemination, such as morphometry, number of motile sperm, curvilinear velocity, average path velocity or straight line velocity, show good correlation with pregnancy rates after IUI (intrauterine insemination) (Macleod & Irvine, 1995; Marshburn *et al.,* 1992), although their use in diagnosis has not been validated. The TDI programme represents, thus, an adequate model to study male subfertility of unknown etiology.

Bearing in mind the state of the art, it seems that an accurate method to predict the fecundity of semen has not yet found. This method would also serve to confirm the results obtained by the other known methods which, as cited above, are not definitive, for example the basic semen analysis including sperm count, motility and morphology.

The success of ICSI (Intracytoplasmic Sperm Injection) for men with male infertility has been one of the reasons for the delay in implementing appropriated markers of sperm function. Any sperm, even those which would not normally have the capacity to fertilize, are injected into oocytes bypassing selection barriers. In order to treat couples with the least invasive treatment, better prognostic tests for fertile sperm, fertile oocytes and viable embryos are needed to improve the outcome of the treatment and obtain higher pregnancy rates. The findings of the present invention contribute to the search and selection of the most valuable gene markers potentially useful as additional tools for predicting and/or improving the success of assisted reproduction techniques leading to increase the pregnancy outcome of said techniques. The present invention shows an expression fingerprint (profile) related to the fecundity ability of sperm that could complement the semen analysis carried out by conventional fertility tests. By means of the method of the present invention it is possible to select those samples from donor semen banks with appropriate conditions to be used for assisted reproduction techniques, as well as giving realistic information about the chances of success of conjugal assisted reproduction techniques to those couples with unexplained infertility.

## DESCRIPTION OF THE INVENTION

The present invention is focused on the evaluation and assessment of the sperm expression gene profile, in order to define a specific group of genes whose expression is indicative of the fecundity ability of spermatozoa. The expression values of this group of genes could be used as biomarkers to predict fecundity ability of men with normal semen parameters, therefore complementing the results obtained by the other known fertility assessment methods which, as cited above, are not definitive.

In spite of having the normal parameters of semen quality, differences in the fecundity of donor sperm are observed after insemination therapies (Marshburn *et al.,* 1992). Classical sperm variables at the time of baseline evaluation of donors have limited value to predict their reproductive fitness. Thus, close supervision of the clinical results obtained for each donor is the only pragmatic way to discard those who show poor pregnancy rates after a reasonable number of insemination cycles (Johnston *et al.,* 1994). Thus, the aim of the present invention is to define a specific gene expression profile able to reflect the fecundity ability of a given semen donor with better efficiency than classical or conventional semen quality parameters.

To address this issue, a cohort of semen donors with a good semen quality and with a detailed record of reproductive outcome preferably using IUI insemination in different female receptors, were studied. Recruitment of semen donors was done among young university students, who had unknown fertility status at the time of donation, thus being representative of the normozoospermic general population. Therefore, the method of the invention is suitable to investigate the molecular features of unexplained male infertility, because it circumvents some of the shortcomings present in infertile couples, such as the confusing role of the significant proportion of female causes that also contribute to reproductive failure.

Therefore the first step of the present invention is focused on searching and finding out the most valuable gene markers, among an initial panel of 87 target genes described to be expressed in human spermatozoa (Ostermeier *et al.,* 2002; Zhao *et al.,* 2006), which can be efficiently used for predicting and/or improving the pregnancy outcome. Consequently, a molecular classifier of the fertility status of semen donors, preferably for IUI, based on gene expression profiles of sperm has been developed in the present invention. The panel of biomarker genes was selected in a training series of semen donors with a detailed record of IUI reproductive outcome in different female receptors, thus obviating the confounding role of female to reproductive failure. The potential of the gene panel of the present invention as a predictive classifier was validated in an independent series of donors.

As may be seen in the examples below, it was found that the gene expression profile of the genes *EIF5A, RPL13, RPL23A, RPS27A, RPS3, RPS8* and *TOMM7,* either taken separately or in combination, can be efficiently used as a predictive tool for discriminating samples with worst pregnancy rate (PR), showing better results than that obtained from the combination of traditional / conventional sperm quality parameters. In a particularly preferred embodiment of the present invention, a multiplex model was defined with the group of four genes consisting of: *EIF5A, RPL13, RPL23A* and *RPS27A,* selected from the group of seven genes cited above.

Therefore, a molecular classifier of the fertility status of semen donors, particularly for IUI, may be developed based on the expression fingerprint of the above cited seven genes, particularly based on the expression profile of said selection group of four genes. Overall, this model has 97% sensitivity and 90% specifity as a predictive tool for discriminating samples with IUI pregnancy rate <13.6%, far better than that obtained from the assessment bases on the combination of traditional sperm quality parameters. Moreover, the method of the invention could complement the semen conventional analysis of well known fertility tests, in order to select those samples from donor semen banks with appropriate conditions to be used for assisted reproduction, as well as to suggest either a conjugal or a donor IUI in those couples with unexplained infertility.

Sensitivity and specificity are statistical measures of the performance of a binary classification test, also known in statistics as classification function. Sensitivity measures the proportion of actual positives which are correctly identified as such. Specificity measures the proportion of negatives which are correctly identified. A theoretical, optimal prediction should reach 100% sensitivity and 100% specificity. So, the method of the invention showing 97% sensitivity and 90% specificity should be considered as a strong and solid tool for predicting fecundity ability of spermatozoa.

Real time PCR amplification in 384-well TaqMan® Low Density Arrays (TLDAs) was considered as an appropriate method for implementing the present invention and for future potential diagnosis purposes, because it allows the simultaneous quantitative amplification of 48 reactions with a minimum cDNA material and a reduced variability due to pipetting. Moreover, in order to improve experimental accuracy, data were normalized to suitable reference genes, which showed constitutive and stable expression levels in the samples investigated.

Therefore the first embodiment of the present invention refers to an *in vitro* method for predicting fecundity ability of spermatozoa comprising the mRNA expression profile analysis of at least one of the following seven genes: *EIF5A, RPL13, RPL23A, RPS27A, RPS3, RPS8* or *TOMM7,* or combinations thereof. In a preferred embodiment the method of the invention comprises the expression profile analysis of a selection group of genes comprising at least one of the following four genes: *EIF5A, RPL13, RPL23A* or *RPS27A,* or combinations thereof. Moreover, in a particularly preferred embodiment, the present invention comprises the mRNA expression profile analysis of the group of genes consisting of: *EIF5A, RPL13, RPL23A* and *RPS27A.*

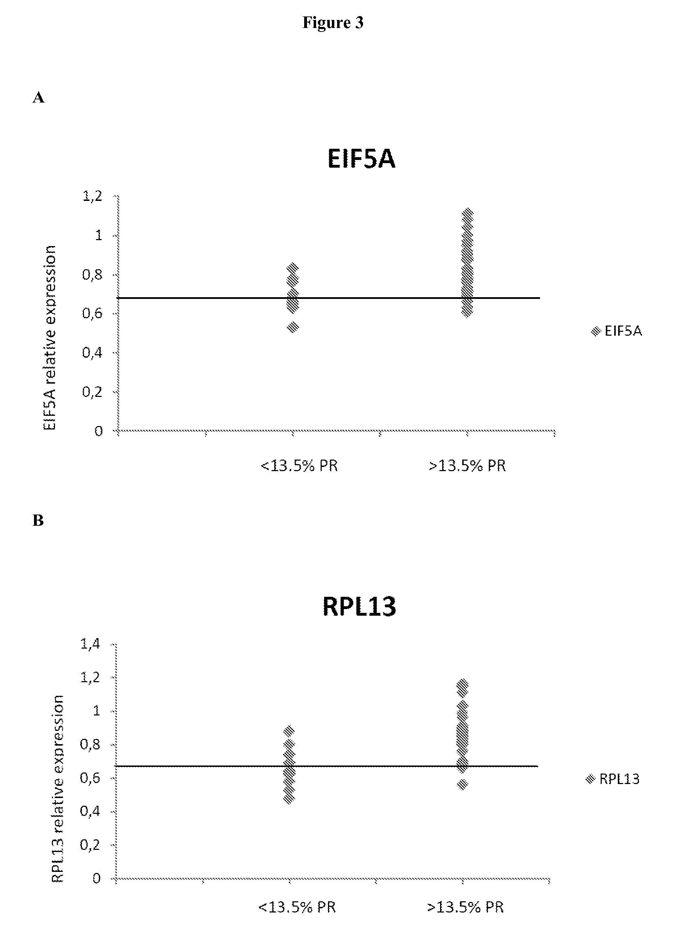

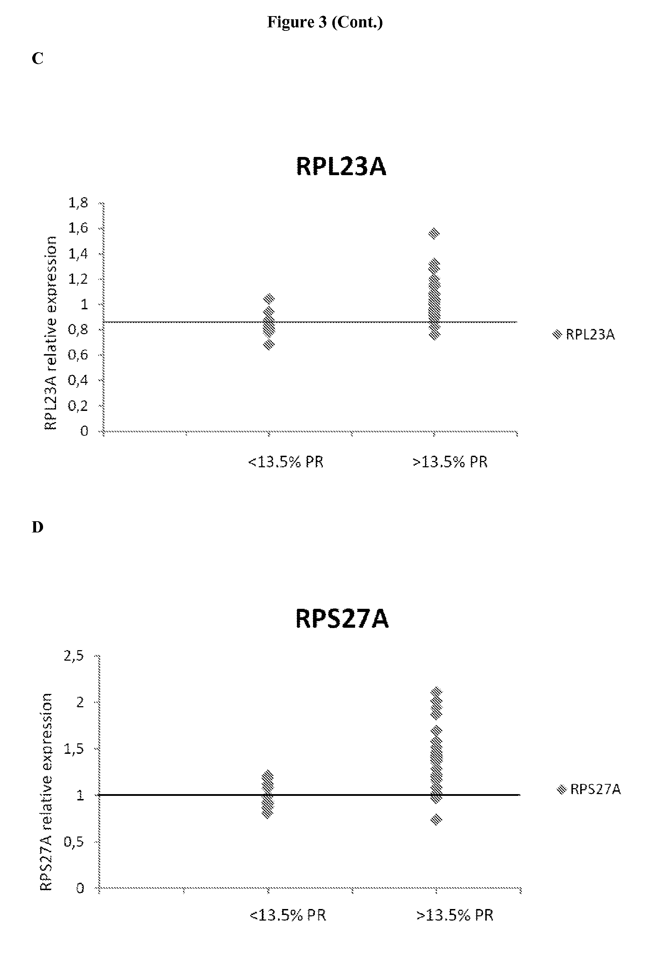

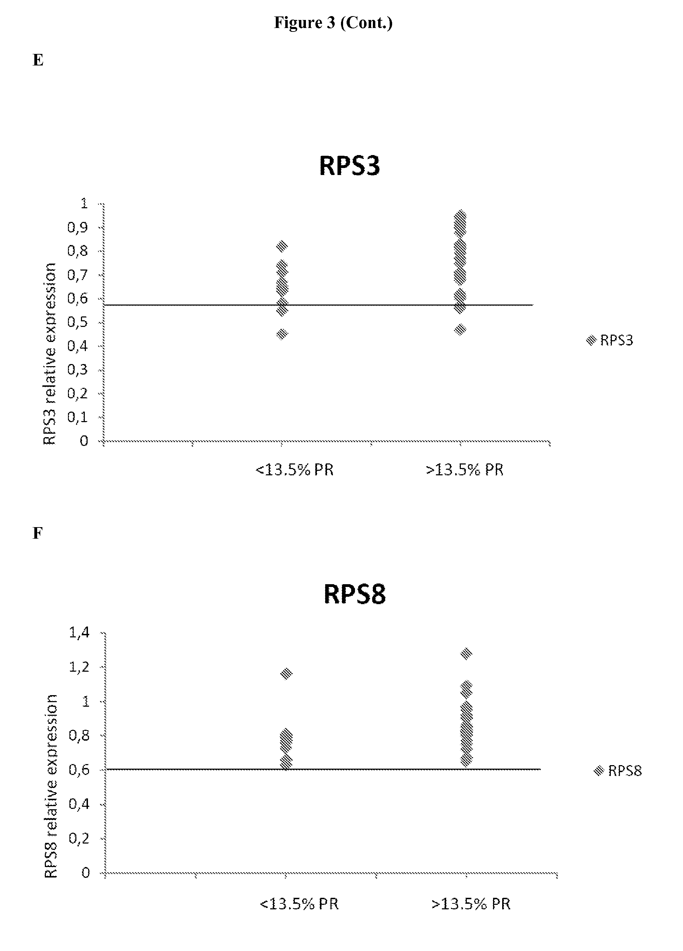

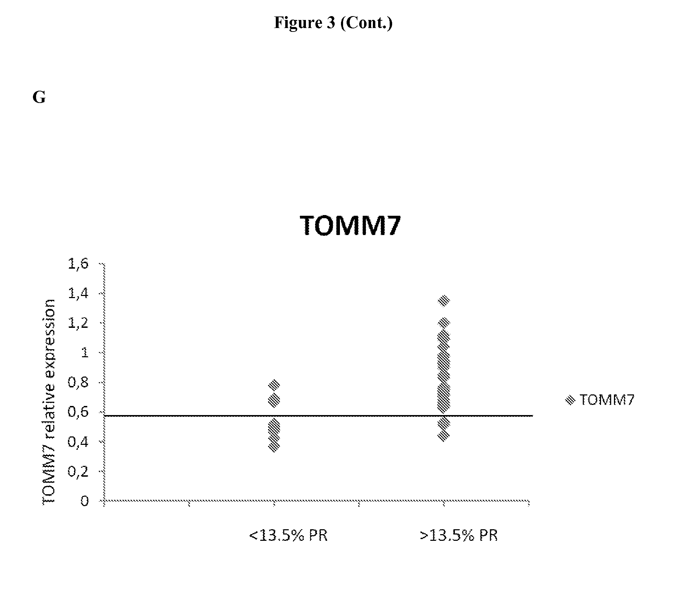

The method of the invention is carried out taking into account that the genes *EIF5A, RPL13, RPL23A, RPS27A, RPS3, RPS8* or *TOMM7* are infra-expressed in samples with low fecundity ability as compared with their expression level in samples with optimal fecundity ability (see **Figure 3**).

The method of the invention is particularly focused on predicting fecundity ability of spermatozoa when intrauterine insemination is selected as fertilization technique. However, it is equally suitable to be used when an alternative reproduction technique is used.

Finally, the method of the invention is able to predict fecundity ability both in semen donors from semen banks and in couples with unexplained infertility. Therefore, the method of the invention could complement the semen conventional analysis, as fertility test, for example in order to select those samples from donor semen banks with appropriate conditions to be used for assisted reproduction as well as to suggest a conjugal or a donor IUI in those couples with unexplained infertility.

Consequently, following the above explained method of the invention it would be possible to predict the pregnancy outcome.

Since the above cited seven genes are well conserved in different species, the method of the invention may be used to predict fecundity ability not only of human spermatozoa but also of animal spermatozoa. The prediction of fecundity of animal spermatozoa is interesting in several fields, for example in order to control the fecundity of animals in danger of extinction.

**BRIEF DESCRIPTION OF THE FIGURES**

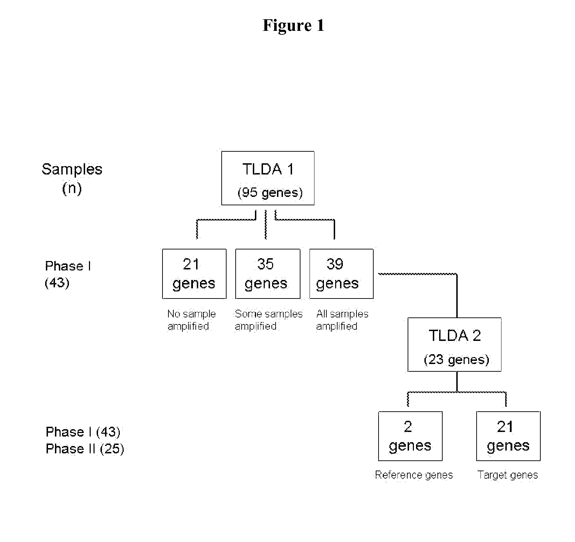

> **Figure 1.** Flow chart representing the number of samples and candidate genes through the stages for phase I (exploring phase) and II (validating phase).

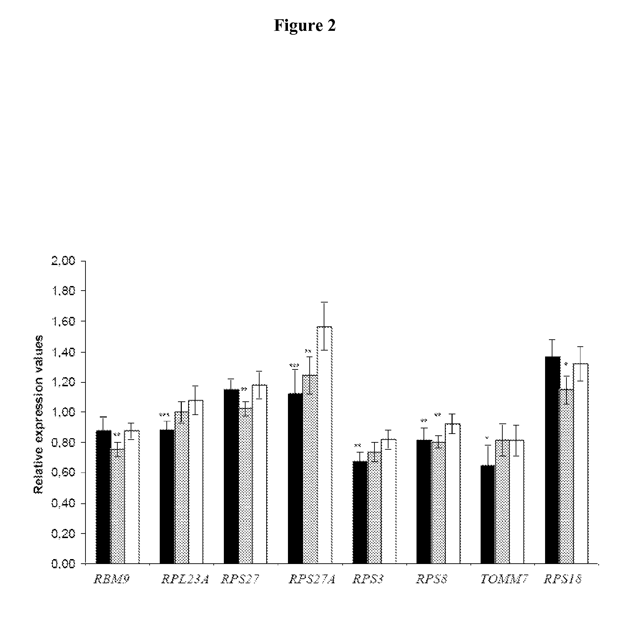

> **Figure 2.** Expression ratios of the differentially expressed genes (Kruskal-Wallis test) in IUI semen samples with worse pregnancy rate (<15.70%, group 1, black bars), intermediate (15.71-23%, group 2, striated bars) and those with better pregnancy rate (>23.01%, group 3, white bars) using *RPS17* as normalizer. Significant differences between group 1 or 2 and group 3 (controls) are indicated by *asterisks* (*, $p \leq 0.05$; **, $p \leq 0.01$; ***, $p \leq 0.001$). Data

with IC 95%.

**Figure 3.** Semen *EIF5A, RPL13, RPL23A, RPS27A, RPS3, RPS8* and *TOMM7* expression ratio as a marker for fecundity of sperm. Horizontal line indicates the transcript ratio threshold value *(EIF5A:* 0.67; *RPL13:* 0.65; *RPL23A:* 0.85; *RPS27A:* 1.01, *RPS3:* 0.58, *RPS8:* 0.60 and *TOMM7:* 0.58) that predicts an IUI pregnancy rate >13.5% (receiver operating characteristic curve analysis). The sensitivity and specificity values are described in **Table 5.**

**Figure 4**.

**A.** Distribution of samples by their IUI pregnancy rate and the predicted probability of the four genetic variables, showing the potential of the model to discriminate samples with worse fertility ability.

**B.** Distribution of samples by their IUI pregnancy rate and the predicted probability of the four clinical variables, showing that this model is unable to discriminate samples with worse fertility ability.

## EXAMPLES

### Example 1. Experimental design

The study was divided into two phases. In the first phase or training phase, a general overview of gene expression behaviour was determined in relation to the pregnancy rates obtained by sperm donors and a gene set expression signature was obtained. In the second phase, the gene set fingerprints was validated as a predictive diagnostic tool in an independent series of donor semen samples. The study was approved by the Institutional Review Board of the Centre.

### Example 2. Selection of semen donors

Cryopreserved semen samples (0.5 ml straws) were procured from the semen bank made up of semen donors referred to the Andrology Service of the Fundació Puigvert and used for TDI from 1994 to 2006. Advertisement for candidate donors was done among universitary students, and most of them had not attempted procreation at the time of recruitment. The clinical procedures for screening semen donors included full personal and familiar medical history to rule out heritable conditions, physical examination and a minimum of two semen tests [performed in accordance with World Health Organization guidelines (WHO, 1999) except for motility assessments, that were done at room temperature]. Spermiograms included volume, pH, sperm concentration, motility, vitality, morphology and antisperm antibodies. Computer assisted sperm analysis (CASA) was performed with a Hamilton-Thorn 2030 system (software version 6.4). Serological tests for HIV I and II, hepatitis B and C, cytomegalovirus and syphilis were done at baseline. At the end of the donations, after six months of quarantine, only donors that tested negative were used. Karyotype was done in donors enrolled after the year 2000.
All semen samples were frozen within two hours after collection, on an equal volume of glycerol-egg-yolk-citrate cryopreservative medium (Sperm Freezing medium, Irvine Scientific, Santa Ana, CA, USA) in liquid nitrogen, using 1.8 mL cryovials, and stored at -196°C until needed. Cryosurvival was assessed as the percent progressive motility of sperm after thawing in a bath at 37°C.

### Example 3. Female receptors and insemination procedures

Women entering into the program of TDI (Therapeutic Donor Insemination) of the Fundació Puigvert that were inseminated with samples of the selected donors were considered for the study. Indications for TDI included severe male factors in the majority of cases, and ejaculatory disturbances or hereditary conditions of the husband. Ovulatory status was studied by biphasic temperature charts and progesterone at midluteal phase, and a normal hysterosalpingography.
In all cases mild follicular stimulation was induced with 75 UI/day of gonadotrophins (Neo-Fertinorm or Pergonal, Serono SA, Spain), and monitored by analysis of estradiol and transvaginal ultrasonography. Ovulation was induced by 10000 UI of HCG (Profasi, HCG Lepori) when at least one follicle of >18 mm was observed. Thawed semen samples (0.5 mL) were diluted with 2 mL of Hams F-10 medium with 0.5% HSA and prepared by differential centrifugation using density gradients (Puregon). Final volume was adjusted to 0.4 mL. Inseminations were done on two consecutive days after 24 and 48 hours after administration of HCG (Human Chorionic Gonadotropin) using an insemination catheter (#4220, Gynétics Medical, Lommel, Belgium). If B-HCG levels were increased 2 to 4 weeks after the inseminations, pregnancy was confirmed by ultrasound scan. Selection of semen donors for insemination was performed by the medical staff on the basis of a matching phenotype of the husband. Semen donor was changed, after 2 or 3 insemination cycles to a particular woman, if pregnancy had not occurred. Donors failing to produce pregnancies were eventually discarded for further use after 20-25 cycles of treatment.

**Example 4. Selection of semen samples**

A total of 68 samples from normozoospermic donors were recruited in the present invention. The inclusion criteria were as follows: having at least 4 surplus frozen aliquots available after the use for insemination purposes, average sperm concentration $\geq 40$ millions/mL; A+B motility $\geq 30\%$, normal morphology $\geq 7\%$ at the time of initial assessment, and >10 insemination cycles per sperm donor.

In the first phase, approximately 2/3 of samples (n=43) were randomly chosen for gene expression analysis (**Figure 1**). Samples were initially classified into three groups (tertiles) that differed in their ability to produce pregnancy after TDI: samples with the worse pregnancy rate (PR) (group 1), intermediate (group 2) and those that achieved the best PR (group 3, as control of a successful PR) (**Table 1**). Semen samples among IUI groups presented similar features on the basis of the concentration, motility and morphology of spermatozoa ($p$= 0.116; $p$=0.814 and $p$=0.936 respectively), as well as, the motility post-thaw of spermatozoa ($p$=0.245) also considered for analysis (**Table 1.A**) (semen conventional parameters). Other clinical factors from male and receptor female related with pregnancy outcome are also indicated (**Table 1.B**).

**Table 1. A**

| | n | Semen volume (mL) | Sperm conc. (million/mL) | Motility (%) | Normal morphology (%) | Motility post-thaw (%) |
|---|---|---|---|---|---|---|
| **IUI (>10 cycles/sample)** | | | | | | |
| Group 1 ($\leq$15.70% pr) | 14 | 3.50 $\pm$ 0.51 | 79.21 $\pm$ 19.28 | 59.17 $\pm$ 5.99 | 23.53 $\pm$ 5.50 | 33.71 $\pm$ 5.52 |
| Group 2 (15.71-23.00% pr) | 14 | 3.82 $\pm$ 0.43 | 101.75 $\pm$ 20.93 | 58.35 $\pm$ 5.41 | 26.30 $\pm$ 6.03 | 38.33 $\pm$ 5.44 |
| Group 3 ($\geq$23.01% pr) | 15 | 3.71$\pm$0.75 | 87.23 $\pm$ 17.62 | 57.06 $\pm$ 5.33 | 24.60 $\pm$ 4.47 | 39.95 $\pm$ 4.62 |
| **Total** | **43** | | | | | |

*IUI: intrauterine insemination.*
*pr: pregnancy rate.*
*n: number of donor samples.*
*conc: concentration.*

**Table 1.B**

| | n | Male age (years) | Male Body Mass index | Receptor women per donor (n) | Female age (years) | Female Body Mass index | Receptor women without GPD per donor (%) | Abortion rate(%) |
|---|---|---|---|---|---|---|---|---|
| **IUI (>10 cycles/sample)** | | | | | | | | |
| Group 1 ($\leq$15.70% pr) | 14 | 24.57 $\pm$ 2.53 | 23.03 $\pm$ 1.29 | 14.50 $\pm$ 4.37 | 34.69 $\pm$ 0.91 | 25.13 $\pm$ 1.07 | 72.64 $\pm$ 6.95 | 12.56 $\pm$ 14.20 |
| Group 2 (15.71-23.00% pr) | 14 | 25.71$\pm$ 2.63 | 22.93 $\pm$ 1.25 | 14.29 $\pm$ 3.40 | 35.40 $\pm$ 0.75 | 24.64 $\pm$ 0.68 | 76.21 $\pm$ 7.12 | 11.58 $\pm$ 6.95 |
| Group 3 ($\geq$23.01% pr) | 15 | 25.53 $\pm$ 2.63 | 23.23 $\pm$ 1.25 | 12.93 $\pm$ 2.70 | 34.79 $\pm$ 0.53 | 25.39 $\pm$ 0.80 | 75.93 $\pm$ 5.85 | 15.63 $\pm$ 5.66 |

(continued)

|  | n | Male age (years) | Male Body Mass index | Receptor women per donor (n) | Female age (years) | Female Body Mass index | Receptor women without GPD per donor (%) | Abortion rate(%) |
|---|---|---|---|---|---|---|---|---|
| IUI (>10 cycles/sample) | | | | | | | | |
| Total | 43 | | | | | | | |
| *IUI: intrauterine insemination.* <br> *pr: pregnancy rate.* <br> *n: number of donor samples.* <br> *GPD: gyneocological pathology diagnosis.* | | | | | | | | |

In the second phase, gene expression fingerprint was validated as a predictive diagnostic tool on 25 additional samples (**Figure 1**).

## Example 5. RNA extraction and cDNA synthesis

In order to enrich fertile spermatozoa and remove somatic contaminants from gene expression analysis of sperm, the frozen-thawed semen samples were individually purified by centrifugation through discontinuous Percoll gradient (65%-90%). Such centrifugation technique is routinely used in ART and relies on the fractioning of cells according to their velocity of sedimentation (Gandini *et al.,* 1999). Semen samples (0.5 mL) were layered on top of the 65% gradient layer and further processed by centrifugation at 175 × g for 30 minutes. This results in the formation of a pellet fraction largely constituted by sperm with normal morphology, and in a supernatant interphase fraction enriched in defective germ cells (sperm with morphological abnormalities, as well as other immature cell types) (Chen & Bongso, 1999; Gil-Guzman *et al.,* 2001). The obtained pellet was resuspended in supplemented HamF10 medium and processed again by centrifugation at 400 × g for 5 minutes.

Total RNA was obtained from the Percoll-purified spermatozoa using NucleoSpin® RNA II Kit (Macherey-Nagel, Duren, Germany), according to the instructions provided by the manufacturer with minor modifications. Briefly, lysis buffer was added to the samples at 600 $\mu$l/$10^7$ cells. The lysates were homogenized with a 20-gauge needle and heated for 30 min at 60 °C. Then it was proceeded to step 4 of the kit, including a DNAse digestion step. RNA was eluted in 40$\mu$l of RNAse-free water. Sample purity and RNA integrity was assessed by reverse-transcription (RT)-PCR using *PRM2* primers as previously described (Ostermeier *et al.,* 2005).

For gene expression study, single-stranded cDNA was obtained by RT of 200 ng of RNA, using random primers and MultiScribe™ Reverse Transcriptase from the High Capacity cDNA Reverse Transcription Kit (AB, Foster City, California, USA) at 37°C for 120 min. Two independent RT reactions were performed from each RNA sample. The resulting cDNA solution was stored at -20°C until use.

## Example 6. Gene expression quantification

Quantitative real-time PCR assays were performed by PCR arrays on micro fluidic cards (MFC), using 384-well TaqMan® Low Density Arrays (TLDAs) on an Applied Biosystems 7900HT Fast Real-Time PCR System (AB, Foster City, California, USA). Half of RT-reaction was applied on each port, each one connecting to 48 reaction wells. A first approach (TLDA1) (**Figure 1**) was performed on the 96-gene format MFC (95 experimental assays and 1 TLDA amplification control) allowing simultaneous measurement of 87 target genes that were selected based on human spermatozoa cell location from cDNA microarrays (Ostermeier *et al.,* 2002; Zhao *et al.,* 2006), and 8 ubiquitously expressed genes, as potential reference genes. Genes and the corresponding assays on demand used for the setup of the TLDA are listed in table 2.

**Table 2**

| Gene Symbol | Gene Name | Assay ID | Role |
|---|---|---|---|
| **PRM-1+** | Protamine-1 | Hs00358158_g1 | Nuclear condensation |
| **PRM-2+** | Protamine-2 | Hs00172518_m1 | Nuclear condensation |

(continued)

| Gene Symbol | Gene Name | Assay ID | Role |
|---|---|---|---|
| **RERE+** | arginine-glutamic acid dipeptide (RE) repeats | Hs00201558_m1 | Transcriptional regulation |
| **FOXG1/FOXG1B +** | Forck head box G1B | Hs01850784_s1 | Transcription regulation. (Early embryo patterning) |
| **TEAD1/TEF1** | TEA domain family member 1 (SV40 transcriptional enhancer factor). Transcriptional enhancer factor TEF-1 | Hs00744253_s1 | Transcription regulation. (Early embryo patterning) |
| **RBM9+** | RNA binding motif protein 9 | Hs00329214_s1 | Transcriptional regulation (RNA binding related) |
| LRRFIP1 | Leucine rich repeat interacting protein 1 | Hs00190993_m1 | Transcriptional regulation (RNA binding related) |
| IREB2 | Iron-responsive element binding protein 2 | Hs00386293_m1 | Transcriptional and translational regulation (RNA and protein binding related) |
| EIF3G | Eukaryotic translation initiation factor 3 delta subunit | Hs00186772_m1 | Translational regulation, initiation |
| **EIF3J/EIF3SI** | Eukaryotic translation initiation factor 3, subunit J (subunit I) | Hs00825842_g1 | Translational regulation, initiation |
| EIF3M/GA17 | Eukaryotic translation initiation factor 3, subunit M | Hs00272235_m1 | Translational regulation, initiation. Fusogenic protein (Sperm-oocyte interaction?) |
| **EIF5A+** | Eukaryotic translation initiation factor 5A | Hs00744729_s1 | Translational regulation, initiation |
| EIF5 | Eukaryotic translation initiation factor 5 | Hs00820472_m1 | Translational regulation, initiation |
| **RPS3+** | Ribosomal protein S3 | Hs02385124_g1 | Protein biosynthesis (ribosomal protein). DNA repair |
| **RPS6+** | Ribosomal protein S6 | Hs02339423_g1 | Protein biosynthesis (ribosomal protein) |
| **RPS8+** | Ribosomal protein S8 | Hs01374307_g1 | Protein biosynthesis (ribosomal protein) |
| **RPS13+** | ribosomal protein S13 | Hs01945436_u1 | Protein biosynthesis (ribosomal protein) |
| **RPS16** | Ribosomal protein S16 | Hs01598518_gH | Protein biosynthesis (ribosomal protein) |
| **RPS17#** | Ribosomal protein S17 | Hs00734303_g1 | Protein biosynthesis (ribosomal protein) |
| **RPS18+** | Ribosomal protein S18 | Hs02387368_g1 | Protein biosynthesis (ribosomal protein) |
| **RPS26** | Ribosomal protein S26 | Hs00955682_g1 | Protein biosynthesis (ribosomal protein) |
| **RPS27+** | Ribosomal protein S27 | Hs01378332_g1 | Protein biosynthesis (ribosomal protein) |

(continued)

| Gene Symbol | Gene Name | Assay ID | Role |
|---|---|---|---|
| **RPS27A / S27a+** | Ribosomal protein S27a | Hs01923841_uH | Protein biosynthesis (ribosomal protein) |
| **RPS29+** | Ribosomal protein S29 | Hs03004310_g1 | Protein biosynthesis (ribosomal protein) |
| **RPL4+** | Ribosomal protein L4 | Hs03044647_g1 | Protein biosynthesis (ribosomal protein) |
| **RPL5** | Ribosomal protein L5 | Hs00851991_u1 | Protein biosynthesis (ribosomal protein) |
| **RPL7+** | Ribosomal protein L7 | Hs02596927_g1 | Protein biosynthesis (ribosomal protein) |
| **RPL10A+** | Ribosomal protein L10a | Hs01912344_uH | Protein biosynthesis (ribosomal protein) |
| **RPL13+** | Ribosomal protein L13 | Hs00761672_s1 | Protein biosynthesis (ribosomal protein) |
| **RPL17** | Ribosomal protein L17, transcript variant 2 | Hs01597859_m1 | Protein biosynthesis (ribosomal protein) |
| **RPL17** | Ribosomal protein L17, transcript variant 1 | Hs00748900_s1 | Protein biosynthesis (ribosomal protein) |
| **RPL23A+** | Ribosomal protein L23a | Hs01921329_g1 | Protein biosynthesis (ribosomal protein) |
| **RPL24** | Ribosomal protein L24 | Hs02338570_gH | Protein biosynthesis (ribosomal protein) |
| **RPL27A** | Ribosomal protein L27a | Hs00741143_s1 | Protein biosynthesis (ribosomal protein) |
| **RPL29#** | Ribosomal protein L29 | Hs00988959_gH | Protein biosynthesis (ribosomal protein) |
| **RPL30** | Ribosomal protein L30 | Hs00265497_ml | Protein biosynthesis (ribosomal protein) |
| **RPL35** | Ribosomal protein L35 | Hs00855441_gH | Protein biosynthesis (ribosomal protein) |
| **RPLP2** | Ribosomal protein, large, P2 | Hs01115130_g1 | Protein biosynthesis (ribosomal protein) |
| **FAU** | Finkel-Biskis-Reilly murine sarcoma virus (FBR-MuSV) ubiquitously expressed | Hs00609872_g1 | Protein biosynthesis (ribosomal protein) |
| RSL1D1 | Ribosomal L1 domain containing 1 | Hs00378363_g1 | Protein biosynthesis (ribosomal protein) |
| **EEF2** | Eukaryotic translation elongation factor 2 | Hs01012839_g1 | Protein biosynthesis (ribosomal protein) |
| RPS4Y1 | 40S ribosomal protein S4, Y isoform (Y-linked 1) | Hs00606158_m1 | Protein biosynthesis (ribosomal protein) |
| MRPL40 | Mitochondrial ribosomal protein L40 | Hs00186843_m1 | Protein biosynthesis (mitochondrial ribosomal protein) |

(continued)

| Gene Symbol | Gene Name | Assay ID | Role |
|---|---|---|---|
| **MRPS18B** | Mitochondrial ribosomal protein S18B | Hs00204096_m1 | Protein biosynthesis (mitochondrial ribosomal protein) |
| **FARSB** | Phenylalanyl-tRNA synthetase beta chain cytoplasmic | Hs00271714_m1 | Protein biosynthesis (cytoplasmic protein) |
| **COPS5** | COP9 constitutive photomorphogenic homolog subunit 5 (Arabidopsis) | Hs00272789_m1 | Protein biosynthesis (nuclear protein) |
| **RPS6KA2** | Ribosomal protein S6 kinase | Hs00179731_m1 | Protein amino acid modification (phosphorylation) |
| ST6GALNAC4 | ST6 (alpha-N-acetyl-neuraminyl-2,3-beta-galactosyl-1,3)-N-acetylgalactosaminide alpha-2,6-sialyltransferase 4 | Hs00205241_m1 | Protein amino acid modification (glycosilation in Golgi apparatus) |
| NARS | Asparaginyl-tRNA synthetase | Hs00189846_m1 | Protein binding related (aspartyl-tRNA aminoacylation) |
| **QARS** | Glutaminyl-tRNA synthetase | Hs00192530_m1 | Protein binding related (glutaminyl-tRNA aminoacylation) |
| **<u>UBC</u>** | Ubiquitin C | Hs00824723_m1 | Protein amino acid modification (Ubiquitin conjugating system) |
| TMED2/RNP24 | Transmembrane emp24 domain trafficking protein 2 / Homo sapiens coated vesicle membrane protein | Hs00607277_m1 | Protein transportation (cytoplasm protein) |
| SCAMP1 | Secretory carrier membrane protein 1 | Hs00792736_m1 | Protein transportation (cytoplasm protein) |
| **<u>VTI1B</u>** | Vesicle transport through interaction with t-SNAREs homolog 1B (yeast) | Hs00762282_s1 | Protein transportation (cytoplasm protein) |
| SLC29A2 | Solute carrier family 29 (nucleoside transporters), member 2 | Hs00155426_m1 | Protein transportation (cytoplasm protein) |
| SLC25A19 | Solute carrier family 25 (mitochondrial thiamine pyrophosphate carrier), member 19, variant 2 | Hs00222265_m1 | Protein transportation (mytochondrial protein) |
| SLC25A25 | Solute carrier family 25 (mitochondrial carrier; phosphate carrier), member 25 | Hs01595832_m1 | Protein transportation (mytochondrial protein) |
| **SLC25A39** | Solute carrier family 25, member 39 | Hs00255405_m1 | Protein transportation (mytochondrial protein) |
| **<u>TOMM7+</u>** | Translocase of outer mitochondrial membrane 7 homolog (yeast) | Hs01628668_s1 | Protein transportation (mytochondrial protein) |
| SFXN3 | Sideroflexin 3 | Hs00229616_m1 | Protein transportation (mytochondrial protein) |
| **CSE1L** | CSE1 chromosome segregation 1-like (yeast) | Hs00354853_m1 | Protein-nucleus import (nuclear pore complex) |

(continued)

| Gene Symbol | Gene Name | Assay ID | Role |
|---|---|---|---|
| **IPO5** | Importin 5 | Hs00267008_m1 | Protein-nucleus import (nuclear pore complex) |
| **XPO1** | Exportin 1 (CRM1 homolog, yeast) | Hs00185645_m1 | Protein-nucleus import (nuclear pore complex) |
| **XPO7** | Exportin 7 | Hs00209262_m1 | Protein-nucleus import (nuclear pore complex) |
| **KPNA2** | Karyopherin alpha 2 (RAG cohort 1, importin alpha 1) | Hs00818252_g1 | Protein-nucleus import (nuclear pore complex) |
| **RANBP2** | RAN binding protein 2 | Hs00397898_g1 | Protein-nucleus import (nuclear pore complex) |
| **PDIA3** | Protein disulfide isomerase family A, member 3 | Hs00607126_m1 | Protein folding (endoplasmic reticulum) |
| WBSCR21/ABHD11 | Abhydrolase domain containing 11 | Hs00541033_g1 | Catalytic activity (metabolic feature of spermatozoa?) |
| **RNF144B/ IBRDC2** | Ring finger 144B / IBR damin containing 2 | Hs00403456_m1 | Catalytic activity, protein ubiquitination (metabolic feature of spermatozoa?) |
| <u>**CCNB1IP1**</u> | Cyclin B1 interacting protein 1 | Hs00820463_g1 | Catalytic activity (metabolic feature of spermatozoa?) |
| <u>**ENO1+**</u> | Enolase 1 (alpha) | Hs00361415_m1 | Catalytic activity (metabolic feature of spermatozoa?) |
| **COX5B** | Cytochrome c oxidase subunit Vb | Hs00426948_m1 | Catalytic activity (metabolic feature of spermatozoa?) |
| FADS1 | Fatty acid desaturase 1 | Hs00203685_m1 | Catalytic activity (metabolic feature of spermatozoa?) |
| <u>**AKAP-4**</u> | A kinase (PRKA) anchor protein 4 | Hs00275849_m1 | Signal transduction. Involved in sperm motility (fertilization and activation of oocyte) |
| FGFR1 | Fibroblast growth factor receptor 1 | Hs00241111_m1 | Signal transduction (spermatogenesis and fertilization?) |
| **TM4SF6** | Tetraspanin 6 | Hs00170288_m1 | Signal transduction (spermatogenesis and fertilization?) |
| GRIN2C | Glutamate receptor, ionotropic, N-methyl D-aspartate 2C | Hs01016626_m1 | Signal transduction (spermatogenesis and fertilization?) |
| **IL6ST** | Interleukin 6 signal transducer | Hs00174360_m1 | Signal transduction (spermatogenesis and fertilization?) |

(continued)

| Gene Symbol | Gene Name | Assay ID | Role |
|---|---|---|---|
| **VAV2** | Vav 2 oncogene | Hs00610104_m1 | Signal transduction (spermatogenesis and fertilization?) |
| WNT5A | Wingless-type MMTV integration site family, member 5A | Hs00998537_m1 | Signal transduction. Embryonic development, Cellular differentiation and morphometric patterning |
| **HLA-E** | Major histocompatibility complex, class 1, E | Hs00428366_m1 | Signal transduction. Immune response protein Signal transducer protein (spermatogenesis and fertilization?) |
| **eNOS/NOS3** | Endothelial nitric oxide synthase, nitric oxide synthase 3 (endothelial cell) | Hs00167166_m1 | Capacitation |
| nNOS/NOS1 | Neuronal nitric oxide synthase, nitric oxide synthase 1 (neuronal) | Hs00167223_m1 | Capacitation |
| **CLGN** | Calmegin precursor variant 1 | Hs00189073_m1 | Testis-specific endoplasmic reticulum chaperone protein. (Sperm-egg interaction, Fertilization) |
| <u>CLU</u> | Clusterin | Hs00156548_m1 | Protein binding. Cell-cell interations; cellular processes for embryo development |
| PTHLH | Parathyroid hormone-like hormone | Hs00174969_m1 | Regulation of gene expression. Hormonal activity. (Pregnancy) |
| CRHBP | Corticotropin releasing hormone binding protein | Hs00181810_m1 | Signal transducer protein. Hormonal activity. (Pregnancy) |
| **HPRT** | Hypoxanthine phosphoribosyltransferase 1 (Lesch-Nyhan syndrome) | Hs99999909_m1 | |
| HMBS | Hydroxymethylbilane synthase | Hs00609297_ml | |
| **PPIA** | Cyclophilin A, peptidylprolyl isomerase A | Hs99999904_m1 | |
| **PGM1** | Phosphoglucomutase 1 | Hs00160062_m1 | |
| GUSB | Glucuronidase, beta | Hs99999908_m1 | |
| PGK1 | Phosphoglycerate kinase 1 | Hs99999906_m1 | |
| **TBP** | TATA box binding protein | Hs00427620_m1 | |
| **KIAA0999/ L19** | KIAA0999 protein | Hs00228549_m1 | |

Gene symbols in **bold** and <u>underlined</u> depict those genes that showed positive PCR-amplifications in all samples. Those genes included in TLDA2 are indicated with + (target genes) or # (reference genes) symbols.

A subsequent second approach (TLDA2) (**Figure 1**) was performed on the 24-gene format MFC, which included 21 target genes, 2 reference genes and an amplification control (**Table 2**). TLDA2 comprised a different RT reaction of the same sample on TLDA1 approach.

Patient and control group samples were always analysed as paired samples in the same analytical run in order to exclude between-run variations. Real-time PCR data were pre-processed and stored in SDS 2.2 software (AB, Foster City, California, USA).

To confirm reproducibility and precision of real-time PCR experiments, inter-assay variation of samples amplified on both approaches were determined. Variation was measured as the coefficient of variation (CV) of Ct from the Ct mean value of both TLDA approaches. In the above mentioned RT-PCR runs, inter-assay variation ranged from 0.63% to 1.60% with the exception of *PRM1* (2.40%), *PRM2* (2.04%), *ENOI* (4.13%) and *RERE* (3.42%), confirming high reproducibility and precision for most of the 23 genes included in the TLDA1 and TLDA2 approaches.

**Example 7. Data analysis**

All statistical analyses were performed using the SPSS version 12 (Lead Technologies, Chicago, USA) software.

The nonparametric Kruskal-Wallis test was first used to analyze the differences in clinical data among IUI study groups of phase I.

Selection of the reference gene/s, showing the most stable expression and the lowest variation among samples, was calculated with both the GeNorm (Vandesompele *et al.,* 2002) and the SPSS version 12 (Lead Technologies, Chicago, USA) programs.

Differences in absolute expression levels of reference genes among study groups 1, 2 and 3 were analyzed by the Kruskal-Wallis test. In order to select the target genes to be included in TLDA2 experiment, differences in absolute expression of TLDA1 target genes in group 1 compared to group 3 were evaluated by the nonparametric Mann-Whitney U test.

Raw data normalization was performed with the qBase program (Hellemans *et al.,* 2007) by using one reference gene as well as by applying geometric averaging of two reference genes, in parallel. Relative quantification (RQ) values were expressed using the $2^{-\Delta\Delta Ct}$ method as fold changes in the target gene normalized to the reference gene and related to the expression of a control sample. In the phase I, the mean value of the TLDA1 and TLDA2 normalized $2^{-\Delta\Delta Ct}$ values for each sample were then subjected to evaluation of statistical significance of differential expression among groups. The nonparametric Kruskal-Wallis test was used to analyze the differences in relative expression of target genes among the IUI study groups. Mann-Whitney U test was used to evaluate differences in relative expression of target genes in patient group 1 or 2 compared to group 3.

Pearson product moment correlation coefficients were calculated to determine the correlation between the expression ratios of the target genes and the IUI PR. Receiver operating characteristic (ROC) curve analysis of the relative expression values was used for distinguishing those individuals with PR $\leq$13.6% for IUI. Accuracy was measured as the area under the ROC curve (AUC). The threshold value was determined by Youden's index, calculated as sensitivity plus specificity - 1(Skendzel & Youden, 1970).

Multivariate binary logistic regressions were used for selection of the optimal combination of genes associated with fertilization status of the phase I samples and for validating the combination of genes as a predictive tool in samples of phase II. A backward stepwise (Conditional) method was used to drop insignificant terms. The multivariate regression model included the genes found to significantly distinguish IUI-PR $\leq$ 13.6%. The binary logistic regression model provides the following estimation of the logit function: Logit(p)= B0 + B1X1 +B2X2 +....

where p=P (adequate fertility potential for insemination), Logit(p)=log(p/(1-p))=log(Odds), B=log OR and Xn= the expression value of the selected genes. Therefore, if we use this estimated model as a prediction model, with the standard classification cutoff of 0.5 (that is, we predict an individual as "having adequate fertility potential for insemination" if its estimated fecundations probability is greater than 0.5), we would classify individuals with a positive Logit function estimation as "adequate for insemination" and individuals with negative Logit function estimation as "inadequate for insemination".

Binary logistic regressions of a single genetic variant as well as single/combination of clinical parameters were calculated for comparison of predictive values of the model.

A *p*-value <0.05 was considered significant.

**Example 8. Selection of genes for the TLDA2 approach**

The presence of mRNA for 74 out of the 95 genes of the TLDA1 study (genes in bold **Table 2**) was confirmed by RT-PCR in human ejaculated spermatozoa. The rest of genes (n=21) could not be amplified (Ct value>33) under the conditions of the study suggesting that the transcript levels were underneath the detection threshold of the technique. From the 74 genes amplified, 35 were excluded for further analysis due to poor amplification efficiency across samples (missing expression values >80%). The mRNA levels of genes amplified in all samples of the study (**Table 2**) were further evaluated (n=39) (**Figure 1**).

In order to achieve precise and reliable quantitative expression results of the genes under study, measurement of gene expression by real-time RT-PCR requires at least one proper internal control gene for normalization purposes. None of the eight genes previously described as ubiquitously expressed have quantifiable expression values, so they were excluded as normalizers. Therefore, among the 39 remaining genes, those showing stable expression levels in the samples investigated were selected and used as normalizers or reference genes. For this purpose, Genorm program was used and this program selected the *RPS17* and *RPL29* as the most stably expressed genes (M= 0.038 for both genes). Moreover, it was assessed that these genes were not differentially expressed among the groups of the study (*p*>0.05). These two genes were included in the TLDA2 approach as reference genes.

The selection of target genes included in the TLDA2 approach (**Table 2**) was performed taking into account those genes that presented statistical difference in Ct values between IUI group 1 and 3.

## Example 9. Relative gene expression profile of donor sperm

Once the TLDA1 and TLDA2 Ct data from the phase I samples were obtained, the quantification of the 21 target gene mRNA levels was, thus, expressed as relative transcript levels using *RPS17* as a single reference gene as well as *RPS17* and *RPL29* genes combination as reference value for both TLDA experiments.

When samples were classified taking into account the PR that resulted when used in IUI, a group comprising eight differentially expressed genes were found among the three groups: *RPL23A, RPS27A, RPS8* (*p*≤0.01), *RBM9, RPS27, RPS3, TOMM7* and *RPS18* (*p*≤0.05), when normalized with both, single and combination, of reference genes (**Figure 2**). All of them presented small intra-group standard deviation values (0.08-0.30).

A selection group of genes comprising: *RPL23A, RPS27A, RPS3, RPS8* and *TOMM7* genes showed a significant fold-change decrease in Group 1 of 1.22, 1.39, 1.22, 1.13, and 1.26 respectively, when compared to Group 3 (*p*≤0.05).

As both, single and combination, of reference genes, data normalization resulted in the same statistical result, gene expression data normalized with *RPS17* was subsequently used for giving more simplicity to the model.

## Example 10. Correlation study between gene expression profiles and semen conventional fecundity parameters or pregnancy rates

No significant correlation was found between the sperm concentration or motility semen parameters and the relative mRNA expression levels of any of the 21 genes analysed. However morphology of spermatozoa was found to be positively correlated with *FOXG1* (*r*:0.341; *p*=0.025) and *RPS8* (*r*:0.371;*p*=0.014) transcript levels.

In order to assess whether there is an association between gene expression and the assisted reproduction PR and to confirm whether the results could be of physiological relevance, a correlation study was performed between the normalized gene expression ratios and the PR mean value of the insemination cycles in which the sample was used. Significant positive correlation coefficients were found between IUI PR and the transcription levels of six genes: *RPL23A, RPL4, RPS27A, RPS3, RPS8* and *TOMM7* (*p*<0.05). **Table 3** shows the Pearson correlation coefficients and adjusted p-values (r; p) between the expression ratios of the target genes and the pregnancy rates after assisted reproduction for all the samples analyzed. Correlation between sperm clinical parameters and pregnancy rates are also shown for comparison. Significant differences (p<0.05) are indicated in bold. PR: pregnancy rate. IUI: intrauterine insemination.

**Table 3**

| Gene expression | | | Sperm clinical parameters | | |
|---|---|---|---|---|---|
| | IUI PR | Abortion rate | | IUI PR | Abortion rate |
| RPL23A | 0.467; *p***=0.002** | 0.110; *p*=0.483 | Sperm concentration | 0.032; *p*=0.837 | -0.043; *p*=0.783 |
| RPL4 | 0.419; *p***=0.005** | 0.055; *p*=0.726 | A + B movility | - 0.024; *p*=0.878 | 0.050; *p*=0.751 |
| RPS27A | 0.530; *p***=0.000** | 0.206; *p*=0.185 | Sperm morphology | 0.154; *p*=0.323 | -0.035; *p*=0.826 |
| RPS3 | 0.463; *p***=0.002** | 0.103; *p*=0.509 | | | |
| RPS8 | 0.450; *p***=0.002** | -0.014; *p*=0.930 | | | |
| TOMM7 | 0.308; *p***=0.044** | 0.189; *p*=0.225 | | | |
| RPL10A | 0.283; *p*=0.066 | 0.403; *p***=0.007** | | | |
| RPS6 | 0.086; *p*=0.584 | 0.326; *p***=0.033** | | | |
| RBM9 | 0.145; *p*=0.352 | -0.301; *p***=0.050** | | | |

**EP 2 532 757 B1**

The same type of analysis was performed for other clinical parameters derived from the IUI, such as the viability rate and abortion rate. Three additional genes: *RPL10A, RPS6* and *RBM9* expression values were found to significantly correlate with abortion rate in IUI study ($p \leq 0.05$) **(Table** 3).

It is hypothesized that there is a threshold level of transcripts with the potential of discriminating those samples with the worst ability to fertilize oocyte. We selected as the state variable the 25th percentile of samples that is $\leq 13.6$ % of PR for IUI. The ROC curve analysis of gene expression levels in IUI resulted in good predictive accuracy (AUC>0.720) of the expression values of seven genes: *EIF5A, RPL13, RPL23A, RPS27A, RPS3, RPS8* and *TOMM7* ($p<0.01$). They were selected as potential genetic biomarkers of sperm fertility status. **Table 4** shows a ROC analysis showing the predictive probabilities of clinical and genetic variables for discriminating those samples with worse fertility ability. IUI: pregnancy rates <13.6%. AUC: area under the curve. CI: confidence interval.

**Table 4**

| Variables | AUC | 95% CI | p-value |
|---|---|---|---|
| | | | |
| **IUI study** | | | |
| | | | |
| Semen concentration | 0.588 | 0.363-0.813 | 0.404 |
| Motility | 0.348 | 0.141-0.556 | 0.151 |
| Normal morphology | 0.615 | 0.399-0.831 | 0.275 |
| Motility post-thaw | 0.598 | 0.416-0.781 | 0.350 |
| EIF5A | 0.827 | 0.691-0.964 | **0.002** |
| RPL 13 | 0.859 | 0.724-0.994 | **0.001** |
| RPL23A | 0.882 | 0.752-1.000 | **0.000** |
| RPS27A | 0.894 | 0.798-0.990 | **0.000** |
| RPS3 | 0.788 | 0.642-0.934 | **0.006** |
| RPS8 | 0.770 | 0.586-0.954 | **0.011** |
| TOMM7 | 0.870 | 0.748-0.992 | **0.000** |

Moreover, **Table 5** shows a univariable logistic regression analyses, showing that each of the seven genes comprised in the **Table 5** may be efficiently used to predict fecundity ability of spermatozoa (validated in samples of phase II). The specificity (Sp) and the sensitivity (Sn) for predicting an IUI PR $\leq 13.6\%$ is depicted for each gene.

**Table 5**

| Variables | Phase I | Phase II | p-value |
|---|---|---|---|
| | Sp; Sn | Sp; Sn | |
| | | | |
| **EIF5A** | 50; 91 | 43; 94.5 | 0.008 |
| **RPL13** | 60; 97 | 0; 100 | 0.004 |
| **RPL23A** | 70; 94 | 14; 94.4 | 0.003 |
| **RPS27A** | 60; 91 | 43; 72 | 0.003 |
| **RPS3** | 20; 91 | 14; 94.4 | 0.013 |
| **RPS8** | 0; 100 | 14; 94.4 | 0.119 |
| **TOMM7** | 70; 88 | 28; 83 | 0.004 |

**Example 11. Multiplex model: multivariate logistic regression analysis**

To determine if a multiplex model could improve performance over single biomarkers, the previously selected genetic biomarkers were analyzed in a multivariate regression analysis. A backward stepwise (conditional) method was used to drop insignificant terms from the model in phase I samples.

Referring IUI study, this analysis resulted in a model that included *EIF5A, RPL13, RPL23A* and *RPS27A* genes (**Table 6**). **Table 6** shows multivariate logistic regression analyses of genetic variables compared to clinical variables in the different phases of the study. IUI: intrauterine insemination. SC: sperm concentration. MT: motility. MPh: morphology. MT-C: Motility post-thaw Sp: specificity. Sn: sensibility. OR: odds ratio. CI: confidence interval. Note: For the multivariate analysis, a backward stepwise (Conditional) method was used to drop insignificant terms in phase I samples. The resulting classifier was used in phase II samples for validation.

**Table 6**

| Variables | Phase I | Phase II | OR | 95% CI | p-value |
|---|---|---|---|---|---|
| | Sp; Sn | Sp; Sn | | for OR | |
| **IUI study** | | | | | |
| | | | | | |
| EIF5A + RPL13 + RPL23A + RPS27A | 90; 97 | 71.5; 78 | | | |
| EIF5A | | | 1.176 | 0.918-1.505 | 0.200 |
| RPL13 | | | 0.907 | 0.720-1.143 | 0.410 |
| RPL23A | | | 1.164 | 0.975-1.390 | 0.093 |
| RPS27A | | | 1.113 | 1.018-1.217 | 0.018 |
| | | | | | |
| SC + MT + MPh+ MT-C | 20; 100 | 14; 94 | | | |
| sc | | | 1.018 | 0.987-1.051 | 0.259 |
| MT | | | 0.870 | 0.764-0.990 | 0.035 |
| MPh | | | 1.028 | 0.936-1.129 | 0.560 |
| MT-C | | | 1.095 | 0.987-1.215 | 0.087 |

The sensitivity and the specificity for predicting an IUI PR ≤13.6% were 97% and 90%, respectively. The accuracy of the test was corroborated as the calculated AUC was 0.955 ($p$=0.000) and the $p$-value of Hosmer and Lemeshow test was 0.554. As comparison, the combination of the four clinical values of sperm resulted in a sensitivity of 100% but a specificity of 20% (AUC:0.761; $p$=0.013).

The classifier based on gene expression values of phase I samples was validated in samples of phase II, resulting in a sensitivity of 78% and a specificity of 71.5% (**Table 6**). For this sample distribution the classification of semen donors is predicted by the formula:

$$\text{Log-odds (individual)} = B0 + B1X1 + B2X2 + B3X3 + B4X4$$

If the log-odds of the individual results in a negative number, then the sample is classified as having inadequate fertility potential for IUI, if is positive it is classified as adequate.

**REFERENCES**

Avendano, C., Franchi, A., Jones, E. & Oehninger, S. (2009) Pregnancy-specific {beta}-1-glycoprotein 1 and human leukocyte antigen-E mRNA in human sperm: differential expression in fertile and infertile men and evidence of a possible functional role during early development. Hum Reprod, 24, 270-277.

Bartoov, B., Eltes, F., Pansky, M., Lederman, H., Caspi, E. & Soffer Y. (1993) Estimating fertility potential via semen analysis data. Hum Reprod. 8, 65-70.

Boerke, A., Dieleman, S. J. & Gadella, B. M. (2007) A possible role for sperm RNA in early embryo development. Theriogenology, 68 Suppl 1, S147-155.

Braude, P., Bolton, V. & Moore, S. (1988) Human gene expression first occurs between the four- and eight-cell stages of preimplantation development. Nature, 332, 459-461.

Cooper, T. G., Noonan, E., von Eckardstein, S., Auger, J., Baker, H. W., Behre, H. M., Haugen, T. B., Kruger, T., Wang, C., Mbizvo, M. T. & Vogelsong, K. M. (2010) World Health Organization reference values for human semen characteristics. Hum Reprod Update, 16, 231-245.

Chen, M. J. & Bongso, A. (1999) Comparative evaluation of two density gradient preparations for sperm separation for medically assisted conception. Hum Reprod, 14, 759-764.

Gandini, L., Lenzi, A., Lombardo, F., Pacifici, R. & Dondero, F. (1999) Immature germ cell separation using a modified discontinuous Percoll gradient technique in human semen. Hum Reprod, 14, 1022-1027.

Garrido, N., Martinez-Conejero, J. A., Jauregui, J., Horcajadas, J. A., Simon, C., Remohi, J. & Meseguer, M. (2009) Microarray analysis in sperm from fertile and infertile men without basic sperm analysis abnormalities reveals a significantly different transcriptome. Fertil Steril, 91, 1307-1310.

Gil-Guzman, E., Ollero, M., Lopez, M. C., Sharma, R. K., Alvarez, J. G., Thomas, A. J., Jr. & Agarwal, A. (2001) Differential production of reactive oxygen species by subsets of human spermatozoa at different stages of maturation. Hum Reprod, 16, 1922-1930.

Guo, X., Gui, Y. T., Tang, A. F., Lu, L. H., Gao, X. & Cai, Z. M. (2007) Differential expression of VASA gene in ejaculated spermatozoa from normozoospermic men and patients with oligozoospermia. Asian J Androl, 9, 339-344.

Gur, Y. & Breitbart, H. (2006) Mammalian sperm translate nuclear-encoded proteins by mitochondrial-type ribosomes. Genes Dev, 20, 411-416.

Guzick, D. S., Overstreet, J. W., Factor-Litvak, P., Brazil, C. K., Nakajima, S. T., Coutifaris, C., Carson, S. A., Cisneros, P., Steinkampf, M. P., Hill, J. A., Xu, D. & Vogel, D. L. (2001) Sperm morphology, motility, and concentration in fertile and infertile men. NEngl J Med, 345, 1388-1393.

Hellemans, J., Mortier, G., De Paepe, A., Speleman, F. & Vandesompele, J. (2007) qBase relative quantification framework and software for management and automated analysis of real-time quantitative PCR data. Genome Biol, 8, R19.

Johnston, R. C., Kovacs, G. T., Lording, D. H. & Baker, H. W. (1994) Correlation of semen variables and pregnancy rates for donor insemination: a 15-year retrospective. Fertil Steril, 61, 355-359.

Krawetz, S. A. (2005) Paternal contribution: new insights and future challenges. Nat Rev Genet, 6, 633-642.

Lewis, S. E. (2007) Is sperm evaluation useful in predicting human fertility? Reproduction, 134, 31-40.

Macleod, I. C. & Irvine, D. S. (1995) The predictive value of computer-assisted semen analysis in the context of a donor insemination programme. Hum Reprod, 10, 580-586.

Marshburn, P. B., McIntire, D., Carr, B. R. & Byrd, W. (1992) Spermatozoal characteristics from fresh and frozen donor semen and their correlation with fertility outcome after intrauterine insemination. Fertil Steril, 58, 179-186.

Miller, D. & Ostermeier, G. C. (2006) Towards a better understanding of RNA carriage by ejaculate spermatozoa. Hum Reprod Update, 12, 757-767.

Miteva, K., Valkov, N., Goncharova-Peinoval, J., Kovachev, K., Zlatarev, S., Pironcheva, G. & Russev, G. (1995) Electron microscopic data for the presence of post-meiotic gene expression in isolated ram sperm chromatin. Cytobios, 83, 85-90.

Naz, R. K. (1998) Effect of actinomycin D and cycloheximide on human sperm function. Arch Androl, 41, 135-142.

Ostermeier, G. C., Dix, D. J., Miller, D., Khatri, P. & Krawetz, S. A. (2002) Spermatozoal RNA profiles of normal fertile men. Lancet, 360, 772-777.

Ostermeier, G. C., Goodrich, R. J., Diamond, M. P., Dix, D. J. & Krawetz, S. A. (2005) Toward using stable spermatozoal RNAs for prognostic assessment of male factor fertility. Fertil Steril, 83, 1687-1694.

Ostermeier, G. C., Miller, D., Huntriss, J. D., Diamond, M. P. & Krawetz, S. A. (2004) Reproductive biology: delivering spermatozoan RNA to the oocyte. Nature, 429, 154.

Platts, A. E., Dix, D. J., Chemes, H. E., Thompson, K. E., Goodrich, R., Rockett, J. C., Rawe, V. Y., Quintana, S., Diamond, M. P., Strader, L. F. & Krawetz, S. A. (2007) Success and failure in human spermatogenesis as revealed by teratozoospermic RNAs. Hum Mol Genet, 16, 763-773.

Samplaski, M. K., Agarwal, A., Sharma, R. & Sabanegh, E. (2010) New generation of diagnostic tests for infertility: review of specialized semen tests. Int J Urol, 17, 839-847.

Siffroi, J. P. & Dadoune, J. P. (2001) Accumulation of transcripts in the mature human sperm nucleus: implication of the haploid genome in a functional role. Ital J Anat Embryol, 106, 189-197.

Skendzel, L. P. & Youden, W. J. (1970) Systematic versus random error in laboratory surveys. Am J Clin Pathol, 54, 448-450.

Steger, K., Wilhelm, J., Konrad, L., Stalf, T., Greb, R., Diemer, T., Kliesch, S., Bergmann, M. & Weidner, W. (2008) Both protamine-1 to protamine-2 mRNA ratio and Bcl2 mRNA content in testicular spermatids and ejaculated spermatozoa discriminate between fertile and infertile men. Hum Reprod, 23, 11-16.

Vandesompele, J., De Preter, K., Pattyn, F., Poppe, B., Van Roy, N., De Paepe, A. & Speleman, F. (2002) Accurate normalization of real-time quantitative RT-PCR data by geometric averaging of multiple internal control genes. Genome Biol, 3, RESEARCH0034.

World Health Organization (1999) Laboratory Manual for the Examination of Human Semen ans Sperm-Cervical Mucus Interaction, p. Cambridge University Press, New York.

Zhao, Y., Li, Q., Yao, C., Wang, Z., Zhou, Y., Wang, Y., Liu, L., Wang, Y., Wang, L. & Qiao, Z. (2006) Characterization and quantification of mRNA transcripts in ejaculated spermatozoa of fertile men by serial analysis of gene expression. Hum Reprod, 21, 1583-1590.

**Claims**

1. *In vitro* method for predicting fecundity ability of spermatozoa comprising the mRNA expression profile analysis of at least one of the following genes: *EIF5A, RPL13, RPL23A, RPS27A, RPS3, RPS8* or *TOMM7,* or combinations thereof.

2. *In vitro* method, according to claim 1, comprising the mRNA expression profile analysis of at least one of the following genes: *EIF5A, RPL13, RPL23A* or *RPS27A,* or combinations thereof.

3. *In vitro* method, according to claim 1, comprising the mRNA expression profile analysis of the group of genes consisting of: *EIF5A, RPL13, RPL23A* and *RPS27A.*

4. *In vitro* method, according to claims 1 to 3, **characterized in that** the genes *EIF5A, RPL13, RPL23A, RPS27A, RPS3, RPS8* or *TOMM7* are infra-expressed in samples with low fecundity ability as compared with their corre-

19

sponding mRNA expression level thresholds in samples with optimal fecundity ability.

5. *In vitro* method, according to any of the claims 1 to 4, **characterized in that** it is able to predict fecundity ability of spermatozoa when intrauterine insemination is elected as fertilization technique.

6. *In vitro* method, according to any of the claims 1 to 4, **characterized in that** it is able to predict fecundity ability both in semen donors from semen banks and in semen of men from couples with unexplained infertility.

7. *In vitro* method, according to any of the claims 1 to 4, **characterized in that** it is able to predict fecundity ability both in human and animal spermatozoa.

8. *In vitro* method for predicting pregnancy outcome comprising the prediction of fecundity ability of spermatozoa by means of the method of claims 1 to 4.


**Patentansprüche**

1. *In-vitro*-Verfahren zur Vorhersage der Fruchtbarkeit von Samen, das die Analyse des mRNA-Expressionsprofils von mindestens einem der folgenden Gene umfasst: *EIF5A, RPL13, RPL23A, RPS27A, RPS3, RPS8* oder *TOMM7,* oder Kombinationen davon.

2. *In-vitro*-Verfahren nach Anspruch 1, das die Analyse des mRNA-Expressionsprofils von mindestens einem der folgenden Gene umfasst: *EIF5A, RPL13, RPL23A* oder *RPS27A,* oder Kombinationen davon.

3. *In-vitro*-Verfahren nach Anspruch 1, das die Analyse des mRNA-Expressionsprofils der Gruppe von Genen bestehend aus *EIF5A, RPL13, RPL23A* und *RPS27A* umfasst.

4. *In-vitro*-Verfahren nach den Ansprüchen 1 bis 3, **dadurch gekennzeichnet, dass** die Gene *EIF5A, RPL13, RPL23A, RPS27A, RPS3, RPS8* oder *TOMM7* in Proben mit niedriger Fruchtbarkeit im Vergleich zu den entsprechenden mRNA-Expressionsschwellenwerten in Proben mit optimaler Fruchtbarkeit unterexprimiert sind.

5. *In-vitro*-Verfahren nach einem der Ansprüche 1 bis 4, **dadurch gekennzeichnet, dass** es in der Lage ist, die Fruchtbarkeit von Samen vorherzusagen, wenn die intrauterine Insemination als Befruchtungstechnik gewählt wird.

6. *In-vitro*-Verfahren nach einem der Ansprüche 1 bis 4, **dadurch gekennzeichnet, dass** es in der Lage ist, die Fruchtbarkeit sowohl in Samenspendern aus Samenbanken als auch in Samen von Männern aus Paaren mit unerklärter Unfruchtbarkeit vorherzusagen.

7. *In-vitro*-Verfahren nach einem der Ansprüche 1 bis 4, **dadurch gekennzeichnet, dass** es in der Lage ist, die Fruchtbarkeit sowohl in menschlichen als auch in tierischen Samen vorherzusagen.

8. *In-vitro*-Verfahren zur Vorhersage des Schwangerschaftsausgangs, das die Vorhersage der Fruchtbarkeit von Samen mittels des Verfahrens nach den Ansprüchen 1 bis 4 umfasst.


**Revendications**

1. Procédé *in vitro* de prédiction de la fécondité des spermatozoïdes comprenant l'analyse du profil d'expression de l'ARNm d'au moins un des gènes suivants : *EIF5A, RPL13, RPL23A, RPS27A, RPS3, RPS8* ou *TOMM7,* ou leurs combinaisons.

2. Procédé *in vitro,* selon la revendication 1, comprenant l'analyse du profil d'expression de l'ARNm d'au moins un des gènes suivants : *EIF5A, RPL13, RPL23A* ou *RPS27A,* ou leurs combinaisons.

3. Procédé *in vitro,* selon la revendication 1, comprenant l'analyse du profil d'expression de l'ARNm du groupe de gènes composé de : *EIF5A, RPL13, RPL23A* et *RPS27A.*

4. Procédé *in vitro,* selon les revendications 1 à 3, **caractérisé en ce que** les gènes *EIF5A, RPL13, RPL23A, RPS27A,*

*RPS3, RPS8* ou *TOMM7* sont sous-exprimés dans des échantillons avec une basse fécondité en comparaison de leurs seuils de niveau d'expression de l'ARNm correspondants dans des échantillons avec une fécondité optimale.

5. Procédé *in vitro,* selon n'importe laquelle des revendications 1 à 4, **caractérisé en ce qu'**il peut prédire la fécondité des spermatozoïdes lorsqu'une insémination intra-utérine est choisie comme technique de fécondation.

6. Procédé *in vitro,* selon n'importe laquelle des revendications 1 à 4, **caractérisé en ce qu'**il peut prédire la fécondité aussi bien du sperme des donneurs de sperme dans des banques de sperme que du sperme des hommes dans des couples présentant uns stérilité non expliquée.

7. Procédé *in vitro,* selon n'importe laquelle des revendications 1 à 4, **caractérisé en ce qu'**il peut prédire la fécondité des spermatozoïdes qu'ils soient humains ou animaux.

8. Procédé *in vitro* pour prédire l'issue d'une grossesse, comprenant la prédiction de la fécondité des spermatozoïdes au moyen du procédé des revendications 1 à 4.

**Figure 1**

**Figure 2**

**Figure 3**

A

**EIF5A**

B

**RPL13**

## Figure 3 (Cont.)

C

## RPL23A

D

## RPS27A

## Figure 3 (Cont.)

E

F

Figure 3 (Cont.)

G

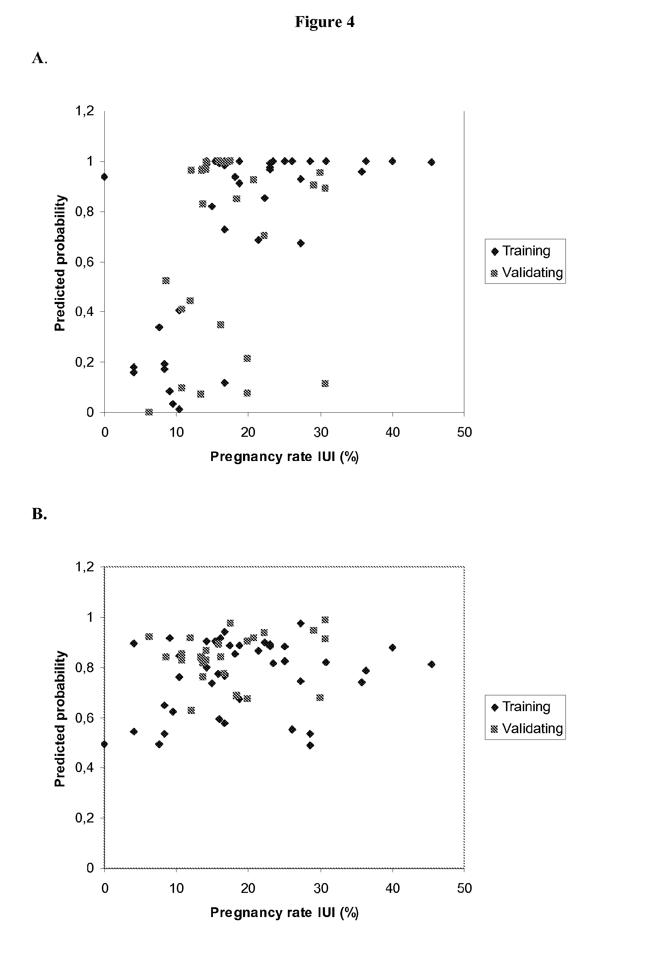

**Figure 4**

A.

B.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **LALANCETTE C. et al.** *Biol Reprod.,* 2008, vol. 78 (4), 618-35
- **FEUGANG JM. et al.** *Reprod Biomed Online,* 2010, vol. 21 (3), 312-24
- **AVENDANO, C. ; FRANCHI, A. ; JONES, E. ; OEHNINGER, S.** Pregnancy-specific {beta}-1-glycoprotein 1 and human leukocyte antigen-E mRNA in human sperm: differential expression in fertile and infertile men and evidence of a possible functional role during early development. *Hum Reprod,* 2009, vol. 24, 270-277
- **BARTOOV, B. ; ELTES, F. ; PANSKY, M. ; LEDERMAN, H. ; CASPI, E. ; SOFFER Y.** Estimating fertility potential via semen analysis data. *Hum Reprod.,* 1993, vol. 8, 65-70
- **BOERKE, A. ; DIELEMAN, S. J. ; GADELLA, B. M.** A possible role for sperm RNA in early embryo development. *Theriogenology,* 2007, vol. 68 (1), 147-155
- **BRAUDE, P. ; BOLTON, V ; MOORE, S.** Human gene expression first occurs between the four- and eight-cell stages of preimplantation development. *Nature,* 1988, vol. 332, 459-461
- **COOPER, T. G. ; NOONAN, E. ; VON ECKARDSTEIN, S. ; AUGER, J. ; BAKER, H. W. ; BEHRE, H. M. ; HAUGEN, T. B. ; KRUGER, T. ; WANG, C. ; MBIZVO, M. T.** World Health Organization reference values for human semen characteristics. *Hum Reprod Update,* 2010, vol. 16, 231-245
- **CHEN, M. J. ; BONGSO, A.** Comparative evaluation of two density gradient preparations for sperm separation for medically assisted conception. *Hum Reprod,* 1999, vol. 14, 759-764
- **GANDINI, L. ; LENZI, A. ; LOMBARDO, F. ; PACIFICI, R. ; DONDERO, F.** Immature germ cell separation using a modified discontinuous Percoll gradient technique in human semen. *Hum Reprod,* 1999, vol. 14, 1022-1027
- **GARRIDO, N. ; MARTINEZ-CONEJERO, J. A. ; JAUREGUI, J. ; HORCAJADAS, J. A. ; SIMON, C. ; REMOHI, J. ; MESEGUER, M.** Microarray analysis in sperm from fertile and infertile men without basic sperm analysis abnormalities reveals a significantly different transcriptome. *Fertil Steril,* 2009, vol. 91, 1307-1310

- **GIL-GUZMAN, E. ; OLLERO, M. ; LOPEZ, M. C. ; SHARMA, R. K. ; ALVAREZ, J. G. ; THOMAS, A. J., JR. ; AGARWAL, A.** Differential production of reactive oxygen species by subsets of human spermatozoa at different stages of maturation. *Hum Reprod,* 2001, vol. 16, 1922-1930
- **GUO, X. ; GUI, Y. T. ; TANG, A. F. ; LU, L. H. ; GAO, X. ; CAI, Z. M.** Differential expression of VASA gene in ejaculated spermatozoa from normozoospermic men and patients with oligozoospermia. *Asian J Androl,* 2007, vol. 9, 339-344
- **GUR, Y. ; BREITBART, H.** Mammalian sperm translate nuclear-encoded proteins by mitochondrial-type ribosomes. *Genes Dev,* 2006, vol. 20, 411-416
- **GUZICK, D. S. ; OVERSTREET, J. W. ; FACTOR-LITVAK, P. ; BRAZIL, C. K. ; NAKAJIMA, S. T. ; COUTIFARIS, C. ; CARSON, S. A. ; CISNEROS, P. ; STEINKAMPF, M. P. ; HILL, J. A.** Sperm morphology, motility, and concentration in fertile and infertile men. *NEngl J Med,* 2001, vol. 345, 1388-1393
- **HELLEMANS, J. ; MORTIER, G. ; DE PAEPE, A. ; SPELEMAN, F. ; VANDESOMPELE, J.** qBase relative quantification framework and software for management and automated analysis of real-time quantitative PCR data. *Genome Biol,* 2007, vol. 8, R19
- **JOHNSTON, R. C. ; KOVACS, G. T. ; LORDING, D. H. ; BAKER, H. W.** Correlation of semen variables and pregnancy rates for donor insemination: a 15-year retrospective. *Fertil Steril,* 1994, vol. 61, 355-359
- **KRAWETZ, S. A.** Paternal contribution: new insights and future challenges. *Nat Rev Genet,* 2005, vol. 6, 633-642
- **LEWIS, S. E.** Is sperm evaluation useful in predicting human fertility?. *Reproduction,* 2007, vol. 134, 31-40
- **MACLEOD, I. C. ; IRVINE, D. S.** The predictive value of computer-assisted semen analysis in the context of a donor insemination programme. *Hum Reprod,* 1995, vol. 10, 580-586
- **MARSHBURN, P. B. ; MCINTIRE, D. ; CARR, B. R. ; BYRD, W.** Spermatozoal characteristics from fresh and frozen donor semen and their correlation with fertility outcome after intrauterine insemination. *Fertil Steril,* 1992, vol. 58, 179-186

- **MILLER, D. ; OSTERMEIER, G. C.** Towards a better understanding of RNA carriage by ejaculate spermatozoa. *Hum Reprod Update,* 2006, vol. 12, 757-767
- **MITEVA, K. ; VALKOV, N. ; GONCHAROVA-PEINOVAL, J. ; KOVACHEV, K. ; ZLATAREV, S. ; PIRONCHEVA, G. ; RUSSEV, G.** Electron microscopic data for the presence of post-meiotic gene expression in isolated ram sperm chromatin. *Cytobios,* 1995, vol. 83, 85-90
- **NAZ, R. K.** Effect of actinomycin D and cycloheximide on human sperm function. *Arch Androl,* 1998, vol. 41, 135-142
- **OSTERMEIER, G. C. ; DIX, D. J. ; MILLER, D. ; KHATRI, P ; KRAWETZ, S. A.** Spermatozoal RNA profiles of normal fertile men. *Lancet,* 2002, vol. 360, 772-777
- **OSTERMEIER, G. C. ; GOODRICH, R. J. ; DIAMOND, M. P. ; DIX, D. J. ; KRAWETZ, S. A.** Toward using stable spermatozoal RNAs for prognostic assessment of male factor fertility. *Fertil Steril,* 2005, vol. 83, 1687-1694
- **OSTERMEIER, G. C. ; MILLER, D. ; HUNTRISS, J. D. ; DIAMOND, M. P. ; KRAWETZ, S. A.** Reproductive biology: delivering spermatozoan RNA to the oocyte. *Nature,* 2004, vol. 429, 154
- **PLATTS, A. E. ; DIX, D. J. ; CHEMES, H. E. ; THOMPSON, K. E. ; GOODRICH, R. ; ROCKETT, J. C. ; RAWE, V. Y. ; QUINTANA, S. ; DIAMOND, M. P. ; STRADER, L. F.** Success and failure in human spermatogenesis as revealed by teratozoospermic RNAs. *Hum Mol Genet,* 2007, vol. 16, 763-773
- **SAMPLASKI, M. K. ; AGARWAL, A. ; SHARMA, R. ; SABANEGH, E.** New generation of diagnostic tests for infertility: review of specialized semen tests. *Int J Urol,* 2010, vol. 17, 839-847
- **SIFFROI, J. P ; DADOUNE, J. P.** Accumulation of transcripts in the mature human sperm nucleus: implication of the haploid genome in a functional role. *Ital J Anat Embryol,* 2001, vol. 106, 189-197
- **SKENDZEL, L. P. ; YOUDEN, W. J.** Systematic versus random error in laboratory surveys. *Am J Clin Pathol,* 1970, vol. 54, 448-450
- **STEGER, K. ; WILHELM, J. ; KONRAD, L. ; STALF, T. ; GREB, R. ; DIEMER, T. ; KLIESCH, S. ; BERGMANN, M. ; WEIDNER, W.** Both protamine-1 to protamine-2 mRNA ratio and Bcl2 mRNA content in testicular spermatids and ejaculated spermatozoa discriminate between fertile and infertile men. *Hum Reprod,* 2008, vol. 23, 11-16
- **VANDESOMPELE, J. ; DE PRETER, K. ; PATTYN, F. ; POPPE, B. ; VAN ROY, N. ; DE PAEPE, A. ; SPELEMAN, F.** Accurate normalization of real-time quantitative RT-PCR data by geometric averaging of multiple internal control genes. *Genome Biol,* 2002, vol. 3
- Laboratory Manual for the Examination of Human Semen ans Sperm-Cervical Mucus Interaction. Cambridge University Press, 1999
- **ZHAO, Y. ; LI, Q. ; YAO, C. ; WANG, Z. ; ZHOU, Y. ; WANG, Y. ; LIU, L. ; WANG, Y. ; WANG, L. ; QIAO, Z.** Characterization and quantification of mRNA transcripts in ejaculated spermatozoa of fertile men by serial analysis of gene expression. *Hum Reprod,* 2006, vol. 21, 1583-1590